# EUROPEAN PATENT SPECIFICATION

(11) **EP 2 682 156 B1**
(45) Date of publication and mention of the grant of the patent: **30.08.2017**
(21) Application number: 11860083.2
(22) Date of filing: 03.03.2011
(51) Int. Cl.: A61F 5/00, A61F 7/10

(54) **ANATOMICAL APPLICATOR FOR HEMORRHOIDS**
ANATOMISCHER APPLIKATOR FÜR HÄMORRHOIDEN
APPLICATEUR ANATOMIQUE POUR HÉMORROÏDES

(43) Date of publication of application: 08.01.2014
(73) Proprietor: Martinez Nieto, S.A., 30391 Cartagena (Murcia) (ES)
(72) Inventor: MARTINEZ NIETO, José, Cartagena (Murcia) (ES)
(74) Representative: Temiño Ceniceros, Ignacio
(86) International application number: PCT/ES2011/070138
(87) International publication number: WO 2012/117124

(56) References cited:
- EP-A2- 0 091 405
- WO-A1-84/03434
- ES-U- 1 001 000
- ES-U- 1 001 000
- ES-U- 1 030 501
- ES-U- 1 032 972
- US-A- 3 939 842
- US-A- 5 069 208

## Description

The present invention is related to an anatomical applicator for the treatment and symptomatic relief of external and internal hemorrhoids.

### State of the prior art

Hemorrhoids are painful dilatations of the veins in the lower part of the rectum or anus. Internal hemorrhoids are located above the anal canal and are covered by a mucosa. On the other hand, those external are located below the anorectal union and are covered by outer skin. Among the main symptoms of hemorrhoids are included: (a) anal pain, especially while sitting; (b) anal itching; (c) blood on toilet tissue, stool or in the toilet bowl; (d) pain during bowel movements; and (e) one or more hard tender lumps near the anus.

Around 5% of the population in western countries has symptoms related to hemorrhoids, and the prevalence of the aforementioned symptoms reaches 50% from 50 years of age. It is estimated, approximately, from 50% to 75% of the western population has hemorrhoid symptoms at some point in their lives.

Sedentary occupations, efforts during the work or sport, keep standing for a long time and certain dietary habits are other causes involved in the formation of hemorrhoids.

Hemorrhoids generally appear before the constipation, and this is usually secondary to them, rather than vice versa. Diarrhea, since it increases the anal pressure also increases the clinical incidence of hemorrhoids. Finally, it should be noted that there is often a family history suggesting a background or hereditary predisposition. Pregnancy is the most common cause of hemorrhoids in young women.

A solution widely used in the current state of the art is cryotherapy, i.e. the direct application of cold on the body, and whose main and commonly known effects are the vasoconstriction, the analgesic/anesthetic effect of its application and anti-inflammatory action, since it decreases the arrival of blood to a specific place. The described effects generate a clear and fast relief of inflammation, pain, itching and bleeding caused by hemorrhoids.

Therefore, the objective technical problem that solves the present invention is the development of a device for local symptomatic relief of hemorrhoids, wherein said device is based on the application of techniques of cryotherapy, and being of direct application both for internal hemorrhoids and external hemorrhoids.

Therapeutic ice packs are currently employed for the application of these techniques, which are used for the reduction of swelling, as well as the relief of the pain occurred after the reduction/removal surgery of hemorrhoid or for traumatic injury. Thus, the use of ice packs is not new and has been used extensively in the medical field for this therapeutic treatment.

To date, different cold applicators for the treatment of hemorrhoids are known.

Thus, the document US4331151 describes a device suitable for its introduction through anal, specifically in the anal canal, formed of an essentially cylindrical body, transversely divided into two parts by a central panel extending longitudinally between two upper and lower ends with a coaxial tube with the body between both ends and which is the continuous access of a fluid at a constant temperature, this fluid being subsequently drained. By using a relatively cool fluid, such as cool water, this device may be used to provide a controlled temperature, for extended periods of time.

This document presents as a drawback the need for drainage of the fluid, since this comes in direct contact with the hemorrhoid. On the other hand, it is not clear that this device, by its constitution and geometry allows the simultaneous treatment of internal and external hemorrhoids.

Document EP 0672400 describes a cooling cylindrical device for removal of pain and therapeutic treatment of hemorrhoids and anal fissures comprising a body capable of being inserted into the anus and comprising a series of channels for cold liquid circulation, this being pumped by a pump provided for this purpose or operated by gravity from a container carried by the user.

Obviously, this device is relatively complex since it involves: (i) a peristaltic pump; or (ii) a container carried by the user in an raised position so that some effect of circulation due to gravity is produced.

Document CA1313482 describes a device for the cold application in external and internal hemorrhoids which includes in its interior a certain cooled liquid that can be ice water or a specific gel. This device has a geometry of enlarged head followed by a narrower region as a neck, ending in a region with a enlarged radio again, this region being which receives the cooled liquid through its base, this liquid remaining sealed by means for this purpose.

The device described in the document CA1313482 has a phallic geometry, that is not the most appropriate for use in hemorrhoids, since it requires the dilation of the anus for its introduction, the fact that most of the time is impossible to get. In addition, the introduction of the broad part or head of this device is certainly painful when there is presence of external and internal hemorrhoids, since in the process of the introduction, the head logically will press against hemorrhoids in the narrower area, until finally it can be stayed inside.
EP 0091405 discloses an anatomical applicator comprising a first rectal region that extends perpendicularly from a second base region, the two regions being mutually concentric, wherein said first rectal region is essentially an appendage having a circular cross-section and a diameter that decreases from the base to the upper end; and where second base region has a circular cross-section; and where furthermore, the two regions are hollow, containing a coolant in the free inner space thereof. ES 1001000 U can be considered as the background closest to the invention, it discloses an anatomical applicator defined by the features of the preamble of claim 1. US5069208 discloses a pad for hot or cold therapeutic applications comprising a fluid-tight and flexible covering. US5069208 proposes a transformation temperature, to change from liquid to solid phase, between -15°C and +15°C, specifically disclosing one embodiment with 0°C.

### Explanation of the invention

The anatomical applicator for hemorrhoids aims to a device equipped with a inner solution capable of increasing, due to its anatomical geometry, the effectiveness and applicability of cryotherapy, which is a technique recommended by healthcare specialists in the treatment of hemorrhoids. Therefore, the technical problem which the present invention solves is to provide an ergonomic device for the cold application in the relief of symptoms of internal and/or external hemorrhoids.

The anatomical applicator object of the present invention is made of a plastic material, polyethylene in a preferred embodiment, and houses inside a freezable fluid. Said fluid is a eutectic mixture enabling to maintain a temperature range comprised between 7 °C and 13 °C for approximately 10 minutes.

Thus, the object of the present invention is an anatomical geometry device made of polyethylene containing a cold storage medium thanks to the incorporation of a liquid eutectic mixture inside it, which allows to maintain the cryotherapeutic capacity of the product during an optimum effective time for the relief of hemorrhoids in the affected area. This is added to its particular anatomical shape to be adapted to the rectal area of the body.

The effect of reduction of temperature in the condition area facilitates the reduction of fluids in the intracellular compartment in interstitial areas (reducing inflammation by accumulation of fluids) that causes the swelling or edema. The application of cold is an effective technique for the reduction of swelling in that region.

The use of the cold applicator in a localized area reduces the pressure in the affected area and, thus reducing the pain within the first six to twelve hours. Similarly, the applicator helps in constriction and maintenance of capillary vessels to slow or reduce the bleeding from blood vessels cut.

Throughout the description and claims, the word "comprises" and its variations are not intended to exclude other technical features, additives, components or steps. For those skilled in the art, other objects, advantages and characteristics of the invention will emerge in part from the description and in part from the practice of the invention. The following examples and drawings are provided by way of illustration, and are not intended to be limiting of the present invention. Furthermore, the present invention covers all the possible combinations of particular and preferred embodiments herein indicated.

### Brief description of the drawings

- FIG. 1: It shows a plan and elevation view of the anatomical applicator for hemorrhoids object of the present invention.
- FIG. 2: It shows a view of a sequence of use of the anatomical applicator object of the present invention.
- FIG. 3: It shows a graph resulting from the test of anatomical applicator object of the anatomical applicator object of the present invention, wherein the variation of temperature in terms of time is represented in minutes of: (a) the rectal simulation; (b) the appendage of the anatomical applicator, or part introduced in the anus, in °C; and (c) the base of the applicator, in °C

### Detailed explanation of an embodiment

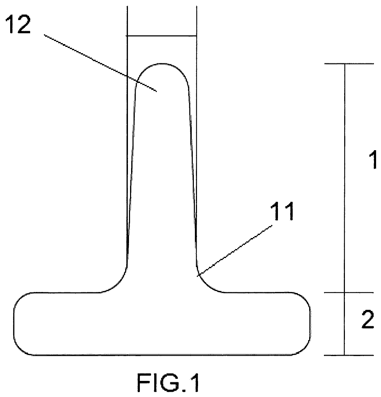

As shown in FIG. 1 the anatomical applicator object of the present invention comprises a first rectal region (1) that extends perpendicularly from a second base region (2), the two regions (1,2) being mutually concentric, the first region (1) has a height comprised between 50 and 60 mm, preferably 55 mm, while the second region (2) has a height comprised between 10 mm and 20 mm, preferably 15 mm; and wherein said first rectal region (1) is essentially an appendage having a circular cross-section and a diameter that decreases from the base (11) to the upper end (12), the diameter of its base (11) being comprised between 13 and 19 mm, preferably 16 mm, the diameter in the upper end (12) being between 1% and 5% less than the diameter of the base (11); and where the second base region (2) has a circular cross-section and a diameter comprised between 65 mm and 75 mm, preferably 70 mm; and where, furthermore, the two regions (1,2) are hollow, containing a eutectic and innocuous mixture in the free inner space thereof, preferably a saline solution said fluid having a liquid-solid phase change at -3°C.

Such eutectic liquid has a cooling temperature which must be achieved by keeping the applicator in the freezer for 2 hours at -18 °C, ranging its operating temperature between 7 °C and 13 °C for a period of between 10 and 15 minutes. In such a way it is provided the cold required to reduce the swelling and relieve the painful symptoms of hemorrhoids, avoiding any risk of burns or cracks produced by the application of ice or an extreme cold. In addition, their anatomical design facilitates its introduction until the stop marked by the base, preventing an introduction higher than that recommended, avoiding accidents or damage to the customer in inner areas of the anus and the rectum.

Thanks to the applicator so described, it is possible to treat internal and external hemorrhoids, facilitating their introduction into the rectal area due to its special configuration, being a unique and simple tool for the treatment and relief of hemorrhoids and, in addition, it can be used easily by the patient without the help of a doctor or healthcare professional.

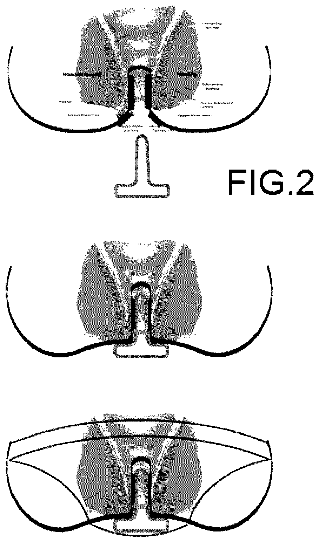

The use of the applicator object of the invention is shown in more detail in FIG. 2. On the other hand, the instructions for use of the applicator lies in keeping the applicator in the freezer, as indicated, until its application, leaving it out around between 1 and 5 minutes prior to use. Due to its anatomical design, once incorporated in the condition area, the applicator is held by underwear, allowing to maintain a normal way of life.

### Results of tests for the anatomical applicator

In the test of the applicator was used one such as the one described, containing an accumulator of heat or eutectic liquid with a temperature of -3 °C, with an approximate latent heat of 73 kcal/Kg, the overall weight of the set being of 52 g and that of the empty applicator of approximately 16 g.

The freezing of samples at temperatures of -18 °C was subsequently carried out in a refrigerator, placing them two type T copper-contantan thermocouples, one in the middle of the first rectal region (1) and other in the second base region (2) near the junction with the first region (1), the thermocouples being fastened with tape, while a third thermocouple was on the "solid water" that be kept at a temperature of 37.5 °C which is the physiological temperature in the rectal area, in a thermally insulated bath. The diameter of the vessel was 100 mm and the height of solid water was 100 mm (vessel of 1000 cm³). Temperatures were measured every 10 seconds with a commercial data acquisition system, brand Yokogawa MW100.

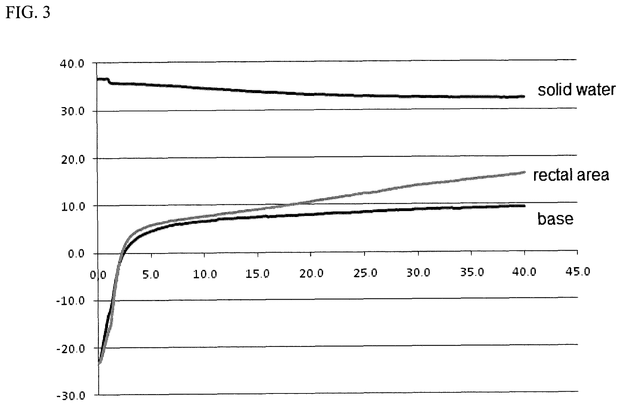

In FIG. 3 it can be seen the variations of temperature in °C, depending on the time in minutes, of the three thermocouples used, wherein the top curve corresponds to the conditions to which the applicator was maintained, i.e. within the "solid water"; the middle to that of the first rectal region (1) or appendage of the applicator and the lower to the base (2) thereof. The time elapsed between the removing from fridge and the introduction in the solid water was 100 seconds, which was equivalent to the necessary to heat the surface at the requested temperature of -6 °C for initiating the application.

In table 1 all the temperature values measured over the testing time have been collected.

**Table 1**

| **Time Min.** | **Temp. of Environment °C** | **Temp. of Base (2) °C** | **Temp. of rectal region (1) °C** |
|---|---|---|---|
| 0.000 | 36.6 | -22.8 | -23.2 |
| 0.167 | 36.7 | -22.7 | -23.0 |
| 0.333 | 36.7 | -20.8 | -21.9 |
| 0.500 | 36.7 | -18.7 | -20.4 |
| 0.667 | 36.6 | -16.7 | -18.9 |
| 0.833 | 36.7 | -14.5 | -17.5 |
| 1.000 | 36.7 | -13.1 | -16.2 |
| 1.167 | 36.0 | -12.1 | -15.2 |
| 1.333 | 35.8 | -10.2 | -12.5 |
| 1.500 | 35.7 | -7.8 | -9.3 |
| 1.667 | 35.7 | -5.6 | -6.5 |
| 1.833 | 35.7 | -3.9 | -4.1 |
| 2.000 | 35.7 | -2.5 | -2.2 |
| 2.167 | 35.6 | -1.5 | -0.6 |
| 2.333 | 35.6 | -0.6 | 0.5 |
| 2.500 | 35.6 | 0.1 | 1.4 |
| 2.667 | 35.6 | 0.7 | 2.2 |
| 2.833 | 35.6 | 1.2 | 2.8 |
| 3.000 | 35.6 | 1.7 | 3.3 |
| 3.167 | 35.6 | 2.1 | 3.7 |
| 3.333 | 35.6 | 2.4 | 4.0 |
| 3.500 | 35.6 | 2.7 | 4.3 |
| 3.667 | 35.5 | 3.0 | 4.6 |
| 3.833 | 35.5 | 3.3 | 4.8 |
| 4.000 | 35.5 | 3.5 | 4.9 |
| 4.167 | 35.5 | 3.7 | 5.1 |
| 4.333 | 35.5 | 3.9 | 5.3 |
| 4.500 | 35.5 | 4.0 | 5.4 |
| 4.667 | 35.4 | 4.2 | 5.6 |
| 4.833 | 35.4 | 4.3 | 5.7 |
| 5.000 | 35.4 | 4.5 | 5.8 |
| 5.167 | 35.4 | 4.6 | 5.9 |
| 5.333 | 35.3 | 4.7 | 6.0 |
| 5.500 | 35.3 | 4.9 | 6.1 |
| 5.667 | 35.3 | 5.0 | 6.2 |
| 5.833 | 35.3 | 5.1 | 6.2 |
| 6.000 | 35.2 | 5.2 | 6.3 |
| 6.167 | 35.2 | 5.3 | 6.4 |
| 6.333 | 35.2 | 5.4 | 6.5 |
| 6.500 | 35.2 | 5.5 | 6.5 |
| 6.667 | 35.1 | 5.6 | 6.6 |
| 6.833 | 35.1 | 5.6 | 6.7 |
| 7.000 | 35.1 | 5.7 | 6.7 |
| 7.167 | 35.0 | 5.8 | 6.7 |
| 7.333 | 35.0 | 5.9 | 6.8 |
| 7.500 | 35.0 | 5.9 | 6.9 |
| 7.667 | 35.0 | 6.0 | 6.9 |
| 7.833 | 35.0 | 5.9 | 7.0 |
| 8.000 | 34.9 | 6.0 | 7.0 |
| 8.167 | 34.9 | 6.1 | 7.1 |
| 8.333 | 34.9 | 6.1 | 7.1 |
| 8.500 | 34.8 | 6.2 | 7.2 |
| 8.667 | 34.8 | 6.2 | 7.2 |
| 8.833 | 34.8 | 6.3 | 7.3 |
| 9.000 | 34.7 | 6.3 | 7.3 |
| 9.167 | 34.7 | 6.3 | 7.4 |
| 9.333 | 34.7 | 6.4 | 7.4 |
| 9.500 | 34.7 | 6.4 | 7.5 |
| 9.667 | 34.6 | 6.5 | 7.5 |
| 9.833 | 34.6 | 6.5 | 7.6 |
| 10.000 | 34.6 | 6.5 | 7.6 |
| 10.167 | 34.5 | 6.5 | 7.7 |
| 10.333 | 34.5 | 6.6 | 7.7 |
| 10.500 | 34.5 | 6.6 | 7.8 |
| 10.667 | 34.5 | 6.7 | 7.8 |
| 10.833 | 34.4 | 6.7 | 7.8 |
| 11.000 | 34.4 | 6.7 | 7.9 |
| 11.167 | 34.4 | 6.8 | 7.9 |
| 11.333 | 34.3 | 6.9 | 8.0 |
| 11.500 | 34.3 | 6.9 | 8.0 |
| 11.667 | 34.3 | 6.9 | 8.1 |
| 11.833 | 34.3 | 7.0 | 8.1 |
| 12.000 | 34.2 | 7.0 | 8.2 |
| 12.167 | 34.2 | 7.0 | 8.2 |
| 12.333 | 34.2 | 7.0 | 8.3 |
| 12.500 | 34.2 | 7.0 | 8.3 |
| 12.667 | 34.1 | 7.0 | 8.3 |
| 12.833 | 34.1 | 7.1 | 8.4 |
| 13.000 | 34.1 | 7.1 | 8.4 |
| 13.167 | 34.1 | 7.2 | 8.5 |
| 13.333 | 34.1 | 7.2 | 8.6 |
| 13.500 | 34.0 | 7.2 | 8.6 |
| 13.667 | 34.0 | 7.2 | 8.6 |
| 13.833 | 34.0 | 7.2 | 8.7 |
| 14.000 | 33.9 | 7.2 | 8.8 |
| 14.167 | 33.9 | 7.2 | 8.8 |
| 14.333 | 33.9 | 7.3 | 8.8 |
| 14.500 | 33.9 | 7.3 | 8.9 |
| 14.667 | 33.8 | 7.3 | 8.9 |
| 14.833 | 33.8 | 7.3 | 9.0 |
| 15.000 | 33.8 | 7.4 | 9.0 |
| 15.167 | 33.8 | 7.4 | 9.1 |
| 15.333 | 33.8 | 7.4 | 9.1 |
| 15.500 | 33.7 | 7.5 | 9.2 |
| 15.667 | 33.7 | 7.5 | 9.2 |
| 15.833 | 33.6 | 7.5 | 9.3 |
| 16.000 | 33.6 | 7.3 | 9.3 |
| 16.167 | 33.6 | 7.4 | 9.3 |
| 16.333 | 33.6 | 7.4 | 9.4 |
| 16.500 | 33.6 | 7.4 | 9.4 |
| 16.667 | 33.6 | 7.4 | 9.5 |
| 16.833 | 33.6 | 7.5 | 9.5 |
| 17.000 | 33.5 | 7.5 | 9.6 |
| 17.167 | 33.5 | 7.5 | 9.6 |
| 17.333 | 33.5 | 7.5 | 9.7 |
| 17.500 | 33.5 | 7.5 | 9.8 |
| 17.667 | 33.5 | 7.6 | 9.8 |
| 17.833 | 33.4 | 7.6 | 9.9 |
| 18.000 | 33.4 | 7.6 | 9.9 |
| 18.167 | 33.4 | 7.6 | 10.0 |
| 18.333 | 33.4 | 7.6 | 10.0 |
| 18.500 | 33.4 | 7.6 | 10.1 |
| 18.667 | 33.3 | 7.6 | 10.1 |
| 18.833 | 33.3 | 7.7 | 10.2 |
| 19.000 | 33.3 | 7.7 | 10.3 |
| 19.167 | 33.3 | 7.7 | 10.3 |
| 19.333 | 33.3 | 7.7 | 10.4 |
| 19.500 | 33.2 | 7.8 | 10.4 |
| 19.667 | 33.2 | 7.8 | 10.5 |
| 19.833 | 33.2 | 7.8 | 10.5 |
| 20.000 | 33.2 | 7.8 | 10.6 |
| 20.167 | 33.2 | 7.8 | 10.7 |
| 20.333 | 33.1 | 7.8 | 10.7 |
| 20.500 | 33.1 | 7.8 | 10.8 |
| 20.667 | 33.1 | 7.9 | 10.8 |
| 20.833 | 33.1 | 7.9 | 10.9 |
| 21.000 | 33.1 | 7.9 | 10.9 |
| 21.167 | 33.1 | 7.9 | 11.0 |
| 21.333 | 33.1 | 8.0 | 11.0 |
| 21.500 | 33.0 | 8.0 | 11.1 |
| 21.667 | 33.1 | 8.0 | 11.2 |
| 21.833 | 33.0 | 8.0 | 11.2 |
| 22.000 | 33.0 | 8.1 | 11.3 |
| 22.167 | 33.0 | 8.1 | 11.3 |
| 22.333 | 33.0 | 8.1 | 11.4 |
| 22.500 | 33.0 | 8.1 | 11.4 |
| 22.667 | 32.9 | 8.1 | 11.5 |
| 22.833 | 32.9 | 8.1 | 11.6 |
| 23.000 | 32.9 | 8.1 | 11.6 |
| 23.167 | 32.9 | 8.1 | 11.7 |
| 23.333 | 32.9 | 8.1 | 11.8 |
| 23.500 | 32.9 | 8.1 | 11.8 |
| 23.667 | 32.9 | 8.1 | 11.8 |
| 23.833 | 32.9 | 8.2 | 11.9 |
| 24.000 | 32.9 | 8.2 | 12.0 |
| 24.167 | 32.8 | 8.2 | 12.1 |
| 24.333 | 32.8 | 8.2 | 12.1 |
| 24.500 | 32.8 | 8.2 | 12.2 |
| 24.667 | 32.8 | 8.3 | 12.2 |
| 24.833 | 32.8 | 8.3 | 12.3 |
| 25.000 | 32.8 | 8.3 | 12.3 |
| 25.167 | 32.8 | 8.3 | 12.3 |
| 25.333 | 32.8 | 8.3 | 12.3 |
| 25.500 | 32.8 | 8.3 | 12.3 |
| 25.667 | 32.7 | 8.3 | 12.4 |
| 25.833 | 32.8 | 8.4 | 12.5 |
| 26.000 | 32.7 | 8.4 | 12.5 |
| 26.167 | 32.7 | 8.4 | 12.5 |
| 26.333 | 32.7 | 8.5 | 12.6 |
| 26.500 | 32.7 | 8.5 | 12.7 |
| 26.667 | 32.7 | 8.5 | 12.8 |
| 26.833 | 32.7 | 8.5 | 12.8 |
| 27.000 | 32.7 | 8.5 | 12.9 |
| 27.167 | 32.7 | 8.6 | 13.0 |
| 27.333 | 32.7 | 8.6 | 13.1 |
| 27.500 | 32.7 | 8.6 | 13.1 |
| 27.667 | 32.7 | 8.6 | 13.2 |
| 27.833 | 32.7 | 8.6 | 13.3 |
| 28.000 | 32.7 | 8.7 | 13.3 |
| 28.167 | 32.7 | 8.6 | 13.4 |
| 28.333 | 32.6 | 8.7 | 13.4 |
| 28.500 | 32.6 | 8.7 | 13.5 |
| 28.667 | 32.6 | 8.7 | 13.6 |
| 28.833 | 32.6 | 8.7 | 13.6 |
| 29.000 | 32.6 | 8.7 | 13.7 |
| 29.167 | 32.6 | 8.7 | 13.7 |
| 29.333 | 32.6 | 8.7 | 13.8 |
| 29.500 | 32.6 | 8.7 | 13.8 |
| 29.667 | 32.6 | 8.8 | 13.9 |
| 29.833 | 32.6 | 8.8 | 13.9 |
| 30.000 | 32.6 | 8.8 | 14.0 |
| 30.167 | 32.6 | 8.8 | 14.0 |
| 30.333 | 32.5 | 8.8 | 14.0 |
| 30.500 | 32.5 | 8.8 | 14.1 |
| 30.667 | 32.5 | 8.8 | 14.1 |
| 30.833 | 32.5 | 8.9 | 14.1 |
| 31.000 | 32.5 | 8.9 | 14.2 |
| 31.167 | 32.5 | 8.9 | 14.2 |
| 31.333 | 32.5 | 8.9 | 14.3 |
| 31.500 | 32.5 | 8.9 | 14.3 |
| 31.667 | 32.5 | 8.9 | 14.4 |
| 31.833 | 32.5 | 9.0 | 14.4 |
| 32.000 | 32.5 | 8.9 | 14.4 |
| 32.167 | 32.5 | 9.0 | 14.5 |
| 32.333 | 32.5 | 9.0 | 14.5 |
| 32.500 | 32.5 | 9.0 | 14.5 |
| 32.667 | 32.5 | 9.0 | 14.6 |
| 32.833 | 32.5 | 9.0 | 14.6 |
| 33.000 | 32.4 | 9.0 | 14.7 |
| 33.167 | 32.5 | 9.1 | 14.7 |
| 33.333 | 32.4 | 8.9 | 14.7 |
| 33.500 | 32.4 | 8.9 | 14.8 |
| 33.667 | 32.4 | 8.9 | 14.8 |
| 33.833 | 32.4 | 8.9 | 14.9 |
| 34.000 | 32.4 | 8.9 | 14.9 |
| 34.167 | 32.4 | 9.0 | 15.0 |
| 34.333 | 32.4 | 9.0 | 15.0 |
| 34.500 | 32.4 | 9.0 | 15.1 |
| 34.667 | 32.4 | 9.0 | 15.1 |
| 34.833 | 32.4 | 9.0 | 15.1 |
| 35.000 | 32.4 | 9.0 | 15.2 |
| 35.167 | 32.4 | 9.1 | 15.2 |
| 35.333 | 32.4 | 9.1 | 15.3 |
| 35.500 | 32.4 | 9.1 | 15.3 |
| 35.667 | 32.4 | 9.1 | 15.4 |
| 35.833 | 32.4 | 9.1 | 15.4 |
| 36.000 | 32.4 | 9.1 | 15.4 |
| 36.167 | 32.4 | 9.1 | 15.5 |
| 36.333 | 32.4 | 9.1 | 15.5 |
| 36.500 | 32.4 | 9.2 | 15.6 |
| 36.667 | 32.4 | 9.2 | 15.6 |
| 36.833 | 32.4 | 9.2 | 15.6 |
| 37.000 | 32.4 | 9.2 | 15.6 |
| 37.167 | 32.4 | 9.2 | 15.7 |
| 37.333 | 32.4 | 9.2 | 15.7 |
| 37.500 | 32.4 | 9.2 | 15.8 |
| 37.667 | 32.4 | 9.3 | 15.8 |
| 37.833 | 32.3 | 9.3 | 15.9 |
| 38.000 | 32.4 | 9.3 | 15.9 |
| 38.167 | 32.4 | 9.3 | 16.0 |
| 38.333 | 32.3 | 9.3 | 16.0 |
| 38.500 | 32.3 | 9.3 | 16.0 |
| 38.667 | 32.3 | 9.3 | 16.1 |
| 38.833 | 32.3 | 9.3 | 16.1 |
| 39.000 | 32.3 | 9.3 | 16.1 |
| 39.167 | 32.3 | 9.3 | 16.1 |
| 39.333 | 32.3 | 9.3 | 16.2 |
| 39.500 | 32.4 | 9.3 | 16.2 |
| 39.667 | 32.4 | 9.3 | 16.3 |
| 39.833 | 32.4 | 9.3 | 16.4 |
| 40.000 | 32.4 | 9.3 | 16.5 |
| 40.167 | 32.4 | 9.3 | 16.5 |
| 40.333 | 32.4 | 9.3 | 16.6 |
| 40.500 | 32.3 | 9.3 | 16.6 |
| 40.667 | 32.4 | 9.4 | 16.7 |
| 40.833 | 32.3 | 9.3 | 16.7 |
| 41.000 | 32.3 | 9.3 | 16.7 |
| 41.167 | 32.3 | 9.3 | 16.8 |
| 41.333 | 32.3 | 9.4 | 16.8 |
| 41.500 | 32.3 | 9.4 | 16.9 |
| 41.667 | 32.3 | 9.4 | 16.9 |
| 41.833 | 32.3 | 9.4 | 16.9 |
| 42.000 | 32.3 | 9.4 | 17.0 |
| 42.167 | 32.3 | 9.4 | 17.0 |
| 42.333 | 32.3 | 9.4 | 17.0 |
| 42.500 | 32.3 | 9.4 | 17.0 |
| 42.667 | 32.3 | 9.5 | 17.1 |
| 42.833 | 32.3 | 9.5 | 17.1 |
| 43.000 | 32.3 | 9.5 | 17.1 |
| 43.167 | 32.3 | 9.5 | 17.2 |
| 43.333 | 32.3 | 9.5 | 17.2 |
| 43.500 | 32.3 | 9.5 | 17.2 |
| 43.667 | 32.3 | 9.5 | 17.2 |
| 43.833 | 32.3 | 9.5 | 17.2 |
| 44.000 | 32.3 | 9.6 | 17.3 |
| 44.167 | 32.3 | 9.6 | 17.3 |
| 44.333 | 32.3 | 9.6 | 17.3 |
| 44.500 | 32.3 | 9.6 | 17.3 |
| 44.667 | 32.3 | 9.6 | 17.4 |
| 44.833 | 32.3 | 9.6 | 17.4 |
| 45.000 | 32.3 | 9.7 | 17.4 |
| 45.167 | 32.3 | 9.7 | 17.4 |
| 45.333 | 32.3 | 9.7 | 17.4 |
| 45.500 | 32.3 | 9.7 | 17.4 |
| 45.667 | 32.3 | 9.7 | 17.4 |
| 45.833 | 32.4 | 9.8 | 17.4 |
| 46.000 | 32.4 | 9.8 | 17.4 |
| 46.167 | 32.3 | 9.8 | 17.5 |
| 46.333 | 32.3 | 9.8 | 17.5 |
| 46.500 | 32.3 | 9.8 | 17.6 |
| 46.667 | 32.3 | 9.9 | 17.6 |
| 46.833 | 32.3 | 9.9 | 17.7 |
| 47.000 | 32.4 | 9.9 | 17.7 |
| 47.167 | 32.4 | 9.9 | 17.7 |
| 47.333 | 32.4 | 10.0 | 17.7 |
| 47.500 | 32.4 | 10.0 | 17.8 |
| 47.667 | 32.4 | 10.0 | 17.8 |
| 47.833 | 32.4 | 10.0 | 17.8 |
| 48.000 | 32.4 | 10.1 | 17.8 |
| 48.167 | 32.4 | 10.1 | 17.9 |
| 48.333 | 32.4 | 9.9 | 17.9 |
| 48.500 | 32.4 | 10.0 | 17.9 |
| 48.667 | 32.4 | 10.1 | 17.9 |
| 48.833 | 32.4 | 10.1 | 17.9 |
| 49.000 | 32.4 | 10.1 | 18.0 |
| 49.167 | 32.4 | 10.1 | 17.9 |
| 49.333 | 32.4 | 10.1 | 17.9 |
| 49.500 | 32.4 | 10.2 | 17.9 |

Several tests with different eutectic liquids were made, suggesting for the intended purpose the use of an accumulator of -3 °C. The accumulator was cooled in refrigerator at the temperature of - 18 °C the accumulator with the appendage (first region (1)) downward and the base (second region (2)) upward.

The product selected for the experimental simulation has been "solid water" which is a highly water absorbent material. In order to keep it warm during the test, assimilating to the rectum, it was placed in a thermally insulated bath at 37.5 °C and held for more than 12 hours.

There were tested different freezing temperatures in the refrigerator, between - 16 °C to - 32 °C, not noticing major differences, since in all the cases is expected to achieve initial introducing temperatures, the same between -6 °C to -4 °C.

It is considered that the anatomical applicator tested, object of tests and studying tests, frozen for more than two hours with the first region (1) downward and applying them within two minutes, can guarantee that the surface temperature in the rectum and surrounding areas, is below 10 °C for 15 minutes.

## Claims

1. Anatomical applicator comprising a first rectal region (1) that extends perpendicularly from a second base region (2), the two regions (1,2) being mutually concentric, said first rectal region (1) is essentially an appendage having a circular cross-section and a diameter that decreases from the base (11) to the upper end (12); and where second base region (2) has a circular cross-section; and where furthermore, the two regions (1,2) are hollow and contain a fluid in the free inner space thereof, **characterized in that:**
said fluid is a eutectic and innocuous fluid having a liquid-solid phase change at -3°C;
the diameter of the second base region (2) is comprised between 65mm and 75mm, preferably 70mm.

2. Applicator according to claim 1 wherein the first region (1) has a height comprised between 50 and 60 mm.

3. Applicator according to claim 2 wherein the first region has a height of 55 mm.

4. Applicator according to claims 1 to 3 wherein the second region (2) has a height comprised between 10 mm and 20 mm.

5. Applicator according to claim 4 wherein the second region (2) has a height of 15 mm.

6. Applicator according to the preceding claims wherein the diameter of the base of the first region (11) is comprised between 13 and 19 mm, preferably 16 mm, the diameter in the upper end (12) being between 1% and 5% less than the diameter of the base (11).

7. Applicator according to the preceding claims wherein the eutectic fluid is a saline solution.

8. Applicator according to the preceding claims wherein, after the applicator was kept in the freezer for 2 hours at -18 °C, the operating temperature of the eutectic fluid ranges between 7 °C and 13 °C for a period of between 10 and 15 minutes, in use.

## Patentansprüche

1. Anatomischer Applikator umfassend einen ersten rektalen Bereich (1), welcher sich senkrecht von einem zweiten Grundbereich (2) erstreckt, wobei die zwei Bereiche (1, 2) gegenseitig konzentrisch sind, wobei der genannte erste rektale Bereich (1) im Wesentlichen ein Ansatz ist, welcher einen Kreisquerschnitt und einen Durchmesser, welcher sich von der Basis (11) bis zum oberen Ende (12) hin verringert, aufweist; und wobei der zweite Grundbereich (2) einen Kreisquerschnitt aufweist; und wobei ferner, die zwei Bereiche (1, 2) hohl sind und eine Flüssigkeit im freien Innenraum derselben enthalten, **dadurch gekennzeichnet, dass**:
die genannte Flüssigkeit eine eutektische und unschädliche Flüssigkeit, welche einen Flüssig-Fest-Phasenübergang bei -3°C aufweist, ist;
der Durchmesser des zweiten Grundbereichs (2) zwischen 65 mm und 75 mm, vorzugsweise bei 70 mm, liegt.

2. Applikator nach Anspruch 1, wobei der erste Bereich (1) eine Höhe, die zwischen 50 und 60 mm liegt, aufweist.

3. Applikator nach Anspruch 2, wobei der erste Bereich eine Höhe von 55 mm aufweist.

4. Applikator nach den Ansprüchen 1 bis 3, wobei der zweite Bereich (2) eine Höhe, die zwischen 10 mm und 20 mm liegt, aufweist.

5. Applikator nach Anspruch 4, wobei der zweite Bereich (2) eine Höhe von 15 mm aufweist.

6. Applikator nach den vorhergehenden Ansprüchen, wobei der Durchmesser der Basis des ersten Bereichs (11) zwischen 13 und 19 mm, vorzugsweise bei 16 mm, liegt, wobei der Durchmesser im oberen Ende (12) zwischen 1% und 5% kleiner als der Durchmesser der Basis (11) ist.

7. Applikator nach den vorhergehenden Ansprüchen, wobei die eutektische Flüssigkeit eine Salzlösung ist.

8. Applikator nach den vorhergehenden Ansprüchen, wobei, nachdem der Applikator im Gefrierfach 2 Stunden bei -18°C gelagert wurde, die Arbeitstemperatur der eutektischen Flüssigkeit bei Verwendung zwischen 7°C und 13°C, während einer Zeitdauer von zwischen 10 und 15 Minuten, schwankt.

## Revendications

1. Applicateur anatomique comprenant une première région rectale (1) qui s'étend perpendiculairement à partir d'une deuxième région de base (2), les deux régions (1, 2) étant mutuellement concentriques, ladite première région rectale (1) est essentiellement un appendice ayant une section transversale circulaire et un diamètre qui se réduit de la base (11) vers l'extrémité supérieure (12) ; et où la deuxième région de base (2) possède une section transversale circulaire; et où, en outre, les deux régions (1, 2) sont creuses et contiennent un fluide dans son espace intérieur libre, **caractérisé en ce que**
ledit fluide est un fluide eutectique et inoffensif ayant un changement de phase liquide-solide à -3°C ;
le diamètre de la deuxième région de base (2) est compris entre 65 mm et 75 mm, de préférence 70 mm.

2. Applicateur selon la revendication 1 dans lequel la première région (1) possède une hauteur comprise entre 50 et 60 mm.

3. Applicateur selon la revendication 2 dans lequel la première région possède une hauteur de 55 mm.

4. Applicateur selon les revendications 1 à 3 dans lequel la deuxième région (2) possède une hauteur comprise entre 10 mm et 20 mm.

5. Applicateur selon la revendication 4 dans lequel la deuxième région (2) possède une hauteur de 15 mm.

6. Applicateur selon les revendications précédentes dans lequel le diamètre de la base de la première région (11) est compris entre 13 et 19 mm, de préférence 16 mm, le diamètre dans l'extrémité supérieure (12) étant entre 1 % et 5% inférieur au diamètre de la base (11).

7. Applicateur selon les revendications précédentes dans lequel le fluide eutectique est une solution saline.

8. Applicateur selon les revendications précédentes dans lequel, après le maintient de l'applicateur dans le congélateur pendant 2 heures à -18°C, la température de travail du fluide eutectique varie lors de son utilisation entre 7°C et 13°C pendant une période d'entre 10 et 15 minutes.
